# EUROPEAN PATENT APPLICATION

(11) **EP 3 929 939 A1**
(43) Date of publication of application: **29.12.2021**
(21) Application number: 20305712.0
(22) Date of filing: 26.06.2020
(51) Int. Cl.: G16H 50/30

(54) **SYSTEM AND METHOD FOR PERI-ANAESTHETIC RISK EVALUATION**

(71) Applicant: Intelligence Anesthesia, 75005 Paris (FR)
(72) Inventor: MOUSSALI, Yassine, 75005 Paris (FR)
(74) Representative: Icosa

(57) **Abstract**

The present invention relates to a peri-anesthesia risk assessment and management system configured to: receive patient data comprising the patient's answers to at least one patient questionnaire, data collected from at least one sensor and data from a patient data repository; from the received patient data, evaluate at least one patient index risk; calculate a global anesthetic risk level based on the at least one patient index risk; select at least one recommendation from a library of recommendations, the at least one recommendation being selected by a perianesthetic assessment algorithm based on the at least one patient index risk and on the global anesthetic risk level; receive as input data comprising information resulting from the user executing the at least one recommendation; modify the at least one patient index risk based on the information resulting from the user executing the at least one recommendation; modify the global anesthetic risk level based on the modified at least one patient index risk.

## Description

### FIELD OF INVENTION

The present invention relates to the field of risk assessment and management in anesthesia.

### BACKGROUND OF INVENTION

Risk assessment and management in anesthesia procedures are fundamental to reduce morbimortality associated with medical procedures conducted under anesthesia. The actual risk assessment is using simple scoring systems, which is not compatible with high accuracy predictive and personalized anesthesia management, which is nowadays possible due to increased accessibility to growing data and healthcare information about patients.

In the context of anesthesia, the American Society of Anesthesiologists (ASA) has developed a classification system to evaluate the anesthetic risk of a patient. The ASA classification is a categorization of a patient's physiological status in 6 categories, therefore this classification system alone does not provide an exhaustive prediction of the peri-operative risk of a patient.

### SUMMARY

The present invention relates to a computer-implemented method for assessing and managing the risks of a patient undergoing an anesthesia procedure, the method comprising the following steps:
a) receiving patient data comprising the patient's answers to at least one patient questionnaire, data collected from at least one sensor and data from a patient data repository;
b) from the received patient data, evaluating at least one patient index risk;
c) calculating a global anesthetic risk level based on the at least one patient index risk;
d) selecting at least one recommendation from a library of recommendations, the at least one recommendation being selected by a perianesthetic assessment algorithm based on the at least one patient index risk and on the global anesthetic risk level;
e) receiving as input data comprising information resulting from the user executing the at least one recommendation;
f) modifying the at least one patient index risk based on the information resulting from the user executing the at least one recommendation;
g) modifying the global anesthetic risk level based on the modified at least one patient index risk.

Contrarily to risk assessment methods in which an anesthetic recommendation is generated based on pre-anesthetic data, such as laboratory test results or pre-existing medical conditions, the present method ensures that the risk assessment is continuously improved based on information resulting from the user executing said recommendation. This information may change the anesthetic management of the patient. Therefore, the present method provides an improved risk assessment and management strategy that is safe and automatized.

In one embodiment, the method further comprises the following steps:
h) periodically, analyzing data in a medical database comprising patient data and measured patient outcomes, so as to calculate correlations between the patient data and the measured patient outcomes;
i) periodically, interrogating a medical guideline database about updated guidelines;
j) modifying or suggesting a modification of the perianesthetic assessment algorithm based on the result of the analysis or the interrogation.

This embodiment allows to improve the perianesthetic assessment and management algorithm based on evidence-based medicine.

The present invention also relates to a computer program product for assessing and managing the risks of a patient undergoing an anesthesia procedure, the computer program product comprising instructions which, when the program is executed by a computer, cause the computer to carry out the steps of the method as described hereabove.

The present invention also relates to a computer-readable storage medium comprising instructions which, when the program is executed by a computer, cause the computer to carry out the steps of the method described hereabove.

The present invention also relates to a peri-anesthesia risk assessment and management system that comprises:
- an acquisition module configured to receive patient data, the patient data comprising: the patient's answers to at least one questionnaire; data collected from at least one sensor and data from a patient data repository;
- a memory configured to store the patient data and a library of recommendations;
- a processing unit for executing a perianesthesia assessment algorithm and an improvement algorithm; the perianesthesia assessment algorithm being configured to:
   - based on the patient data, evaluate at least one patient index risk;
   - evaluate a global anesthetic risk level based on the at least one patient index risk;
   - select at least one recommendation from the library of recommendations based on the at least one patient index risk and on the global anesthetic risk level;
   - receive as input data comprising information resulting from the user executing the at least one recommendation;
   - modify the at least one patient index risk based on the information resulting from the user executing the at least one recommendation;
   - modify the global anesthetic risk level based on the modified at least one patient index risk.
and the improvement algorithm being configured to periodically modify or suggest a modification of the perianesthetic assessment algorithm.

Advantageously, the system allows to automatize the patient management by automatically modifying the risk evaluation based on the information resulting from the user executing the at least one recommendation.

In one embodiment, the system further comprises means for modifying the at least one recommendation selected by the processing unit.

In one embodiment, the memory is further configured to store a reference medical database comprising patient data and patient outcomes collected from a plurality of patients and the improvement algorithm is configured to:
- periodically, analyze data in the reference medical database to identify correlations;
- modify or suggest a modification of the perianesthetic assessment algorithm based on the result of the analysis.

Advantageously, the periodic analysis of the data in the reference medical database allows to ensure the relevance of the recommendations selected by the perianesthetic risk assessment and management algorithm. Thanks to the periodic monitoring of the relevance of the recommendation, and to the modification of the perianesthetic assessment algorithm based on the data analysis, the morbimortality during and after a medical procedure under anesthesia is minimized, thus providing a safe anesthetic assessment and management strategy.

This embodiment also allows to increase the medical knowledge about peri-anesthetic risks by discovering novel correlations and hence, novel index risks.

In one embodiment, the improvement algorithm is further configured to:
- predict a patient outcome based on the patient data;
- receive as input a measured patient outcome;
- compare the predicted patient outcome with the received patient outcome to identify a discrepancy;
- in case of discrepancy, anonymize and store the measured patient outcome and the patient data in a reference medical database.

This embodiment allows to use discrepancies to optimize future predictions.

In one embodiment, the patient outcome and the measured outcomes are selected from a group comprising future admissions or discharges; pre-anesthetic outcomes; early or late post-anesthetic outcomes.

In one embodiment, the improvement algorithm is configured to:
- periodically, interrogate a medical guideline database about updated guidelines;
- modify or suggest a modification of the perianesthetic assessment algorithm based on the result of the interrogation.

Advantageously, this embodiment allows to modify or suggest a modification of the perianesthetic assessment algorithm based on evidence-based medicine, thus improving the quality of the anesthetic assessment and management strategy.

This embodiment also allows to modify the recommendation selection based on changes in the local, national or international guidelines, thereby ensuring that the anesthetic assessment and management strategy is up-to-date.

In one embodiment, the improvement algorithm is a hybrid algorithm.

In one embodiment, the at least one sensor is selected among: an optical sensor, a pressure sensor; a force sensor; a thermal sensor and the data collected from the at least one sensor comprise physiological data or identity data.

In one embodiment, the system according to the present invention further comprises an output module configured to:
- output a first pre-operative anesthesia evaluation comprising the index risk, the global anesthetic risk level and the at least one recommendation;
- output a second pre-operative anesthesia evaluation comprising the selected at least one recommendation; information resulting from the user executing the at least one recommendation; the modified index risk and the modified global anesthetic risk level.

In one embodiment, the at least one patient index risk is selected from a group consisting of: respiratory risk, neurological risk, kidney failure risk, immunologic risk, chronic pain risk, vascular risk, hepatic risk, cardiac risk, airway management risk, nausea and vomiting risk, thromboembolic risk, hemorrhagic risk.

### DEFINITIONS

In the present invention, the following terms have the following meanings:
- **"Peri-anesthesia"** refers to all the phases preceding, and/or during and/or following an anesthesia procedure.
- **-"Semi-automated"** refers to a method, wherein at least one step is computer-implemented and at least one step is manual.

### BRIEF DESCRIPTION OF THE DRAWINGS

The following detailed description will be better understood when read in conjunction with the drawings. For the purpose of illustrating, the system is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

Features and advantages of the invention will become apparent from the following description of embodiments of a system, this description being given merely by way of example and with reference to the appended drawings in which:
F**igure 1** is a block diagram representing one non-limiting example of the steps of a) receiving the patient's answers to a questionnaire 11, patient data 1 collected from sensors 12 and data in a patient data repository 13; b) evaluating at least one patient index risk 2, d) selecting at least one recommendation 4; e) receiving as input data comprising information resulting from the user executing the at least one recommendation 4; f) modifying the at least one patient index risk 2 based on the information resulting from the user executing the at least one recommendation 4; and g) modifying the global anesthetic risk level 3. In this example, four index risks 2 are evaluated; for intelligibility purpose, the aforementioned steps are illustrated for one out of the four index risks 2, notably for the respiratory risk.

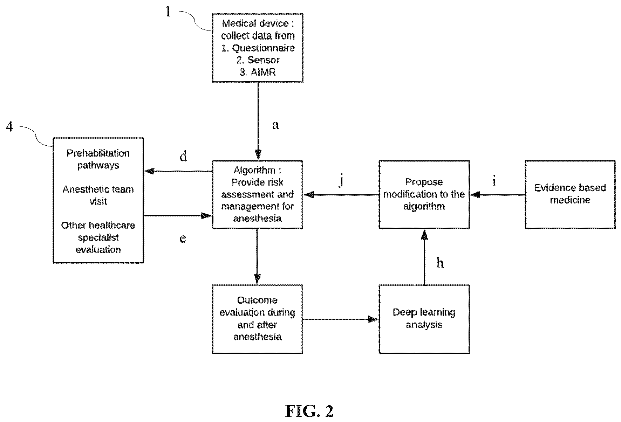

**Figure 2** is a block diagram representing one non-limiting example of the steps performed by the peri-anesthesia risk assessment and management system.

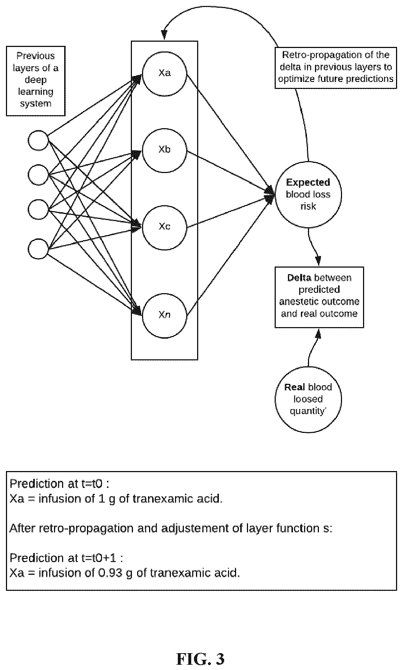

**Figure 3** is a diagram representing one embodiment of the improvement algorithm of peri-anesthesia risk assessment and management system according to the present invention in which the patient outcome is the blood loss and the discrepancy between the predicted patient outcome and the measured patient outcome is the delta between the predicted blood loss and the measured blood volume lost. In the illustrated embodiment, said delta is retro-propagated in the previous layers of the deep learning algorithm, thereby allowing to improve future predictions.

While various embodiments have been described and illustrated, the detailed description is not to be construed as being limited hereto. Various modifications can be made to the embodiments by those skilled in the art without departing from the true spirit and scope of the disclosure as defined by the claims.

### DETAILED DESCRIPTION

The present invention relates to a system and a method to assess and manage the risks of a patient undergoing an anesthesia procedure. In particular, the present invention allows to assess and manage the risks encompassing all the events related to an anesthetic procedure.

The peri-anesthesia risk assessment and management system according to the present invention comprises an acquisition module, a memory and a processing unit.

The acquisition module configured to receive patient data 1 comprising the patient's answers to at least one questionnaire; data in a patient data 1 repository; and data collected from at least one sensor.

In the present invention, the at least one questionnaire comprises:
- a first list of standardized questions;
- a second list of personalized questions based on the patient's answers to the standardized questions;
- a third list of personalized questions based on the patient data collected from the at least one sensor 12.

The second and third lists of questions may be generated by an algorithm executed by the processing unit.

In a preferred embodiment, the patient data repository is an anesthetic information and management record (AIMR) or an electronic medical record (EMR).

The patient data repository may be a repository stored in the memory of the present invention; a repository stored in a database of a healthcare facility, such as for example an AIMR stored in an electronic medical record system of a medical facility.

The patient data repository may a be a repository residing on a storage media of the patient such as the storage unit of his/her smartphone or mobile telephone.

The data 13 from the patient data repository may comprise a scan or a photograph or medical documents in a paper format.

The at least one sensor may comprise: an optical sensor, a pressure sensor; a force sensor; a thermal sensor.

In one embodiment, the at least one sensor is a sensor on a medical device, such as for example a Sp0₂ sensor, an ECG Sensor, a PPG sensor or a sensor of a health-related smart device, such as for example a smartwatch, a fitness tracker wristband, a biopatch, a wearable device.

In one embodiment, the at least one sensor is a sensor of a mobile phone or smartphone, such as for example a camera sensor. Advantageously, this embodiment allows to provide to the perianesthesia system identification data, such as a photograph of the patient or a photograph of a patient's identity document. In addition, the sensor according to this embodiment may provide a photograph of a medical document in paper format.

The patient data 13 from a patient data repository may comprise results of biological screening tests and analysis; medical prescription; genetic profiling; clinical data from patient autonomous auscultation; blood pressure; oxygen saturation and multi-spectrophotometric analysis; arterial pulse wave; electrocardiogram; psychomotricity test result; cardiac stress test result.

The memory is configured to store the patient data 1 and a library of recommendations.

The processing unit is configured to execute a perianesthesia assessment algorithm and an improvement algorithm.

The processing unit may further execute additional algorithms, for example to generate the second and the third list of questions of the patient questionnaire; to predict patient outcomes.

The perianesthesia assessment algorithm is configured to:
- based on the patient data 1, evaluate at least one patient index risk 2;
- evaluate a global risk level 3 based on the at least one patient index risk 2;
- select at least one recommendation 4 from the library of recommendations based on the at least one patient index risk 2 and on the global anesthetic risk level 3;
- receive as input data comprising information resulting from the user executing the at least one recommendation 4;
- modify the at least one patient index risk 2 based on the information resulting from the user executing the at least one recommendation;
- modify the global anesthetic risk level 3 based on the modified at least one patient index risk.

Advantageously, the system allows to automatize the patient management by improving the risk evaluation based on the information resulting from the user executing the at least one recommendation 4.

In one embodiment, the index risk is selected from a group consisting of: respiratory risk, neurological risk, kidney failure risk, hepatic risk, cardiac risk, airway management risk, nausea and vomiting risk, thromboembolic risk, hemorrhagic risk, immunologic risk, chronic pain risk, vascular risk.

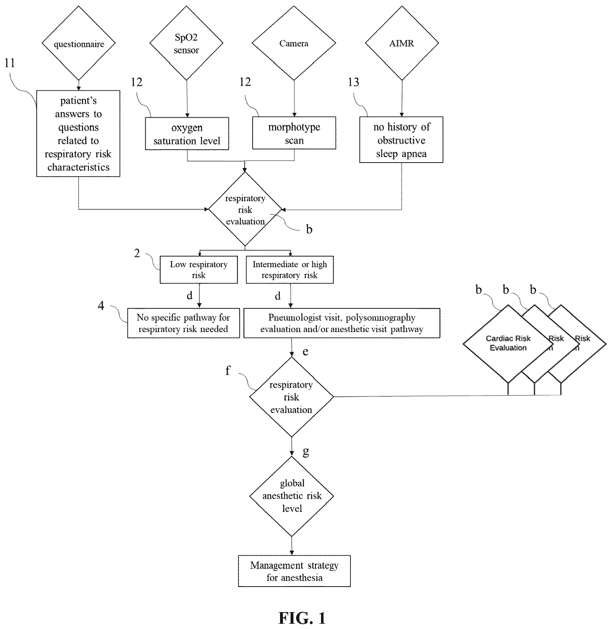

In the embodiment represented in figure 1, the present system evaluates four index risks 2.

In a preferred embodiment, the system of the present invention evaluates a number of index risks 2 comprised between 1 and 15.

One example of this embodiment is represented in figure 1. In this embodiment, the system receives patient data 1 comprising the patient's answers to at least one questionnaire 11; data collected from at least one sensor 12 and data from a patient data repository 13.

In the embodiment illustrated in figure 1, the questionnaire may comprise questions such as for example: « Do you snore loudly? », « Do you often feel tired, fatigued or sleepy during the daytime? »; « Has anyone observed you stop breathing or choking/gasping during your sleep? »; « Are you being treated or have you been treated for high blood pressure? »; « Is your body mass index superior to 35 kg/m²? »; « Are you older than 50 years old? ».

The data collected from at least one sensor 12 may comprise oxygen saturation levels obtained by a SpO2 sensor and a morphotype scan obtained via a camera, as illustrated in figure **1****.**

The data 13 from a patient data repository may comprise data related to a history of obstructive sleep apnea that are stored in an anesthetic information and management record (AIMR), as illustrated in figure **1****.**

In one embodiment, the index risk 2 is a categorical variable; such as for example: low risk, intermediate risk or high risk. One example of this embodiment is represented in figure **1****.**

In one embodiment, the index risk 2 is a continuous variable.

In one embodiment, the global anesthetic risk level is a continuous variable.

In one embodiment, the global anesthetic risk level is a categorical variable.

In one embodiment, the at least one recommendation 4 comprises:
- a nutritional recommendation, such as for example a diet, or a fasting period;
- an activity recommendation, such as a physical exercise;
- an educational recommendation, such as the educational material provided in oral, written, visual or electronic format.

In this embodiment, the information resulting from the user executing the at least one recommendation 4 may comprise the patient weight; the patient's answer to a self-test, the self-test comprising questions configured to assess the patient's understanding of educational material or to assess the patient's psychological status.

For instance, the at least one recommendation 4 may be an educational recommendation, comprising a video configured to inform the patient about regional anaesthesia followed by a self-test configured to assess the patient's psychological status. Advantageously, this embodiment allows to modify the at least one patient index risk 2 and the global anesthetic risk level 3 and hence the anesthetic management strategy, based on the patient's psychological status.

For instance, if the patient autonomously reports a panic attack related to the visualization of the video, or if the patient's answers to the self-test indicate a panic attack episode, the at least one patient index risk 2 and the global anesthetic risk level 3 may be modified accordingly. Because of the panic attack episode, the modified global anesthetic risk level may be higher than the previously calculated global anesthetic risk level 3. As a result, if a regional anesthesia was initially chosen based on the global anesthetic risk level 3, a general anesthesia may replace the initial choice, based on the modified global anesthetic risk level.

In one example of this embodiment, the selected recommendation 4 may be a diet and an objective of weight. In this example, the data comprising information from the user executing the recommendation 4 may be the patient weight. The perianesthesia assessment algorithm receives as input the patient weight and it modifies the at least one patient index risk and the global anesthetic risk level risk based on such information. If the patient weight corresponds to the objective of weight the algorithm may modify the at least one patient index risk and/or the global anesthetic risk level.

In one embodiment, the at least one recommendation 4 comprises a medical pathway. Said medical pathway may comprise one or more anesthesiologic consultations and non-anesthesiologic consultations. The medical pathway is an optimized medical pathway to properly evaluate and manage the anesthetic risk of the patient. In this embodiment, the user executing the at least one recommendation 4 is the anesthesiologist or another healthcare specialist, such as a radiologist, a pneumologist, a cardiologist. In this embodiment, the present recommendation 4 further comprises a consultation modality, said modality being based on the at least one patient index risk 2; and on the global anesthetic risk level 4. For instance, the consultation modality may be an in-person modality or a remote modality.

The information resulting from the user executing the at least one recommendation 4 may comprise information that come to light during a preoperative consultation, such as for example the discovery of a severe aortic stenosis by a cardiologist, or the discovery of a hemophilia by an hematologist, or a severe sleep apnea syndrome by a pneumologist.

In one embodiment, the at least one recommendation 4 comprise the optimal anesthetic protocol for the patient anesthesia procedure, i.e. the protocol that minimize the risk of morbimortality related to the medical procedure performed under anesthesia.

In this embodiment, the information resulting from the user executing the at least one recommendation 4 may comprise per-anesthetic management, such as for example a clinical decision support for optimal antibio-prophylaxis or the cardiac output monitoring to use for one specific patient for a specific surgery, post-anesthetic management such as for example the thrombo-embolic prevention needs, or the modality of blood test monitoring.

In one embodiment, the present invention further comprises means for transmitting the selected recommendation 4 to the patient and to the anesthetic staff.

In one embodiment, the system of the present invention further comprises means for modifying the at least one recommendation 4. The recommendation 4 may be modified by a healthcare provider. This embodiment allows to provide a semi-automated patient management and clinical decision support strategy.

The improvement algorithm is configured to periodically modify or suggest a modification of the perianesthetic risk assessment and management algorithm.

Advantageously, the improvement algorithm ensures that the recommendation 4 selected by the perianesthetic risk assessment and management algorithm is the recommendation 4 that minimizes the morbimortality during and after a medical procedure under anesthesia.

In the present invention, a safe and automatized patient management is obtained by the combination of a rule-based perianesthetic assessment algorithm with a machine-learning-based improvement algorithm.

Advantageously, the improvement algorithm allows to increase the accuracy of the index risk 2 evaluation and the global anesthetic risk level 3 calculation. Therefore, it allows to improve the relevance of the recommendation 4.

In one embodiment, the improvement algorithm is a machine learning or deep learning algorithm.

In one embodiment, the improvement algorithm is a hybrid algorithm, i.e. it comprises a machine learning algorithm and a set of rules.

In one embodiment, the improvement algorithm is configured to:
- periodically, analyze data in a medical database to identify a correlation;
- modify or suggest a modification of the perianesthetic risk and assessment algorithm based on the result of the analysis.

The medical database is a medical database stored in the memory of the system. Said database may comprise data obtained from a healthcare facility.

In one embodiment, medical database comprises a plurality of patient data, each patient data from the plurality is linked to a plurality of patient outcomes. The improvement algorithm may modify or suggest a modification of the perianesthetic assessment algorithm if at least one correlation is above a predefined threshold.

In a preferred embodiment, said medical database is a dynamic database comprising data collected from a plurality of patients evaluated for an anesthetic risk by means of the perianesthetic assessment algorithm of the present invention.

In this embodiment, the system receives at least one measured outcome for each patient evaluated by the perianesthetic assessment algorithm. The measured outcome may be an outcome measured during or after anesthesia, such as for example early clinical outcomes such as walking distance capabilities or quantity of excreted urine during or after the surgery, biological outcomes such as the serum hemoglobin level or serum lactic acid level twenty-four hours after a surgery or late outcomes such as death or chronic pain syndrome. The patient data 1, the patient risk indexes 2; the global anesthetic risk level 3; the at least one recommendation 4; the information related to the execution of the recommendation and the measured patient outcome are stored in the medical database. One example of this embodiment is represented in figure 2.

Preferentially, the medical dataset comprise data collected from more than 100000 patients. Such data comprises patient data 1, such as the answers to the questions of questionnaires 11, data collected from sensors 12 and data stored in patient data repositories 13; and patient outcomes. In this embodiment, the improvement algorithm analyzes said data to identify a correlation between the aforementioned patient data 1 and the patient outcomes.

Advantageously, this embodiment allows to increase the medical knowledge about peri-anesthetic risks by discovering novel correlations.

In one embodiment, the improvement algorithm is configured to:
- periodically, interrogate a medical guideline database about updated guidelines;
- modify or suggest a modification of the perianesthetic assessment algorithm based on the result of the interrogation.

In this embodiment, the improvement algorithm is configured to modify or suggest a modification of the perianesthetic assessment algorithm based on evidence-based medicine. For instance, the modification may be based on the results of a prospective randomized controlled trial or a retrospective study.

Advantageously, this embodiment allows to identify novel index risks 2 related to anesthesia, such as a genetic risk, and automatically add the identified index risk 2 to the other index risks 2 of the perianesthetic assessment algorithm.

In one embodiment, the updatable medical databases comprise anesthetic guidelines database, such as MEDLINE / PubMed; ASA standards and practice guidelines; eGuidelines; Guideline Central; National Institute for Clinical Excellence; National Library for Health (NLH) guidelines database.

In one embodiment, the improvement algorithm is configured to:
- predict a patient outcome based on the patient data 1;
- receive as input a patient outcome;
- compare the predicted patient outcome with the received patient outcome to identify a discrepancy;
- in case of discrepancy, anonymize and store the measured patient outcome and the patient data 1.

In one embodiment, the improvement algorithm is a machine learning model. In this embodiment, if the discrepancy matches the rules in a set of plausibility rules, the patient data 1 and the received patient outcome are added to the training set.

In one embodiment, the improvement algorithm hybrid algorithm which comprises a machine learning algorithm and a rule-based algorithm. In this embodiment, if the discrepancy matches all the rules in the set of plausibility rules, the rules of the predictive model may be modified. Further rules may be added to the predictive model to handle the clinical situation generating the discrepancy.

In one embodiment, the training of the machine learning model comprises calculating a delta related to the discrepancy and training hidden layers through a delta retro-propagation algorithm.

In one embodiment, the validation of the machine learning model comprises: validating the model against a set of consistency rules and validating the model against a set of clinical reference cases.

In one embodiment, the processing unit is further configured to store in the reference medical dataset: the patient index risk 2; the patient global risk level 3; the selected recommendation 4; the data comprising information resulting from the user executing the recommendation 4; the modified patient index risk; the modified global risk level.

This embodiment allows to collect structured data and use them to improve the accuracy of future recommendations 4.

The discrepancy identification also allows identifying an unforeseen complication in the clinical outcomes or a non-adherence of the patient or a healthcare provider to the selected recommendation 4.

In one embodiment, the predicted outcomes and the received outcomes are selected from a group comprising future admissions or discharges; pre-anesthetic outcomes; early or late post-anesthetic outcomes.

One example of this embodiment is represented in figure 3. In this embodiment, the improvement algorithm predicts several variables Xa, Xb, Xc, ... Xn and, based on the predicted variables, it predicts a patient outcome.

In the example illustrated in figure 3, the predicted variables are variables related to a blood loss risk, such as for example a tranexamic acid dosage, and the predicted patient outcome is a blood loss. The algorithm receives as input the measured blood volume lost; a discrepancy between the predicted blood loss and the received blood volume lost is identified, and the delta related to said discrepancy is retro-propagated in the previous layers of the improvement algorithm so as to improve future predictions.

In this particular example, the predicted variable Xa is the amount of tranexamic acid to be infused; the amount predicted by the improvement algorithm is 1 g and the amount predicted after delta retro-propagation is 0.93 g.

In one embodiment, the predicted outcome is selected from a group comprising: information resulting from the user executing the at least one recommendation; a complication, an early post-anesthetic event, a late post-anesthetic event, the need for post-anesthetic medical assessment, the modality of healthcare such as ambulatory or inpatient strategy, the post-anesthetic analgesic, and usual medication adjustment, improved anesthetic management for future anesthesia.

In one embodiment, the system comprises means for modifying the selected recommendation 4. In this embodiment, the processing unit identifies a discrepancy between the selected recommendation 4 and the modified recommendation. Such discrepancy may be anonymized and stored in the medical dataset.

In one embodiment, the system further comprises an output module configured to output a first pre-anesthesia evaluation comprising the global anesthetic risk level; and the selected at least one recommendation. The output module is further configured to output a second pre-anesthesia evaluation comprising the selected at least one recommendation; the information resulting from the user executing the at least one recommendation; the modified global anesthetic risk level.

The present invention also relates to a method to assess and manage the risks of a patient undergoing an anesthesia procedure, the method comprising the following steps:
a) receiving patient data 1 comprising the patient's answers to at least one patient questionnaire 11, data collected from at least one sensor 12 and data from a patient data repository 13;
b) from the received patient data 1, evaluating at least one patient index risk 2;
c) calculating a global anesthetic risk level 3 based on the at least one patient index risk 2;
d) selecting at least one recommendation 4 from a library of recommendations, the at least one recommendation 4 based on the at least one patient index risk 2 and on the global anesthetic risk level 3;
e) receiving as input data comprising information resulting from the user executing the at least one recommendation 4;
f) modifying the at least one patient index risk 2 based on the information resulting from the user executing the at least one recommendation 4;
g) modifying the global anesthetic risk level 3 based on the modified at least one patient index risk.

In one embodiment, the method further comprises repeating the steps a) to g) for a plurality of patients and generating a medical database comprising, for each patient of the plurality, the patient data; the index risk 2; the global anesthetic risk level 3; the selected recommendations 4 and the information resulting from the user executing the at least one recommendation 4.

In one embodiment, one or more steps of the present method are undertaken by a perianesthetic assessment algorithm and the method further comprises:
- periodically, analyzing data in a reference medical database comprising patient data 1 and patient outcomes, so as to identify correlations between at least one of the patient data 1 and a patient outcome;
- periodically, interrogating a medical guideline database about updated guidelines;
- modifying or suggesting a modification of the perianesthetic assessment algorithm based on the result of the analysis or the interrogation.

In one embodiment, the modified perianesthetic assessment algorithm is validated against a set of consistency rules and on a set of clinical reference cases.

The present invention also relates to a computer program product for assessing and managing the risks of a patient undergoing an anesthesia procedure, the computer program product comprising instructions which, when the program is executed by a computer, cause the computer to carry out the steps of the method according to any one of the embodiments described hereabove.

The computer program product to perform the method as described above may be written as computer programs, code segments, instructions or any combination thereof, for individually or collectively instructing or configuring the processor or computer to operate as a machine or special-purpose computer to perform the operations performed by hardware components. In one example, the computer program product includes machine code that is directly executed by a processor or a computer, such as machine code produced by a compiler. In another example, the computer program product includes higher-level code that is executed by a processor or a computer using an interpreter. Programmers of ordinary skill in the art can readily write the instructions or software based on the block diagrams and the flow charts illustrated in the drawings and the corresponding descriptions in the specification, which disclose algorithms for performing the operations of the method as described above.

The present invention further comprises a computer-readable storage medium comprising instructions which, when the program is executed by a computer, cause the computer to carry out the steps of the method according to any one of the embodiments described hereabove.

According to one embodiment, the computer-readable storage medium is a non-transitory computer-readable storage medium.

Computer programs implementing the method of the present embodiments can commonly be distributed to users on a distribution computer-readable storage medium such as, but not limited to, an SD card, an external storage device, a microchip, a flash memory device, a portable hard drive and software websites. From the distribution medium, the computer programs can be copied to a hard disk or a similar intermediate storage medium. The computer programs can be run by loading the computer instructions either from their distribution medium or their intermediate storage medium into the execution memory of the computer, configuring the computer to act in accordance with the method of this invention. All these operations are well- known to those skilled in the art of computer systems.

The instructions or software to control a processor or computer to implement the hardware components and perform the methods as described above, and any associated data, data files, and data structures, are recorded, stored, or fixed in or on one or more non-transitory computer-readable storage media. Examples of a non-transitory computer-readable storage medium include read-only memory (ROM), random-access memory (RAM), flash memory, CD- ROMs, CD- Rs, CD+Rs, CD- RWs, CD+ RWs, DVD-ROMs, DVD- Rs, DVD+Rs, DVD- RWs, DVD+RWs, DVD- RAMs, BD- ROMs, BD- Rs, BD- R LTHs, BD- REs, magnetic tapes, floppy disks, magneto-optical data storage devices, optical data storage devices, hard disks, solid-state disks, and any device known to one of ordinary skill in the art that is capable of storing the instructions or software and any associated data, data files, and data structures in a non-transitory manner and providing the instructions or software and any associated data, data files, and data structures to a processor or computer so that the processor or computer can execute the instructions. In one example, the instructions or software and any associated data, data files, and data structures are distributed over network-coupled computer systems so that the instructions and software and any associated data, data files, and data structures are stored, accessed, and executed in a distributed fashion by the processor or computer.

## Claims

1. A computer-implemented method for assessing and managing the risks of a patient undergoing an anesthesia procedure, the method comprising the following steps:
a) receiving patient data (1) comprising the patient's answers to at least one patient questionnaire (11), data collected from at least one sensor (12) and data from a patient data repository (13);
b) from the received patient data, evaluating at least one patient index risk (2);
c) calculating a global anesthetic risk level (3) based on the at least one patient index risk (2);
d) selecting at least one recommendation (4) from a library of recommendations, the at least one recommendation being selected by a perianesthetic assessment algorithm based on the at least one patient index risk (2) and on the global anesthetic risk level (4);
e) receiving as input data comprising information resulting from the user executing the at least one recommendation (4);
f) modifying the at least one patient index risk (2) based on the information resulting from the user executing the at least one recommendation (4);
g) modifying the global anesthetic risk level (3) based on the modified at least one patient index risk.

2. The method according to claim **1,** further comprising the following steps:
h) periodically, analyzing data in a medical database comprising patient data and measured patient outcomes, so as to calculate correlations between the patient data and the measured patient outcomes;
i) periodically, interrogating a medical guideline database about updated guidelines;
j) modifying or suggesting a modification of the perianesthetic assessment algorithm based on the result of the analysis or the interrogation.

3. A computer program product for assessing and managing the risks of a patient undergoing an anesthesia procedure, the computer program product comprising instructions which, when the program is executed by a computer, cause the computer to carry out the steps of the method according to any of claims **1** or **2.**

4. A computer-readable storage medium comprising instructions which, when the program is executed by a computer, cause the computer to carry out the steps of the method according to any of claims **1** or **2.**

5. A peri-anesthesia risk assessment and management system, the system comprising:
- an acquisition module configured to receive patient data (1) comprising: the patient's answers to at least one questionnaire (11); data collected from at least one sensor (12) and data from a patient data repository;
- a memory configured to store the patient data (1) and a library of recommendations;
- a processing unit for executing a perianesthesia assessment algorithm and an improvement algorithm; the perianesthesia assessment algorithm being configured to:
- based on the patient data (1), evaluate at least one patient index risk (2);
- evaluate a global anesthetic risk level based on the at least one patient index risk (2);
- select at least one recommendation from the library of recommendations based on the at least one patient index risk (2) and on the global anesthetic risk level;
- receive as input data comprising information resulting from the user executing the at least one recommendation;
- modify the at least one patient index risk (2) based on the information resulting from the user executing the at least one recommendation;
- modify the global anesthetic risk level (3) based on the modified at least one patient index risk.
and the improvement algorithm being configured to periodically modify or suggest a modification of the perianesthetic assessment algorithm.

6. The system according to claim **5,** further comprising means for modifying the at least one recommendation (5) selected by the processing unit.

7. The system according to claim **5** or claim **6,** wherein the memory is further configured to store a reference medical database comprising patient data (1) and patient outcomes collected from a plurality of patients and the improvement algorithm is configured to:
- periodically, analyze data in the reference medical database to identify correlations;
- modify or suggest a modification of the perianesthetic assessment algorithm based on the result of the analysis.

8. The system according to any of claims **5** to **7,** wherein the improvement algorithm is further configured to:
- predict a patient outcome based on the patient data (1);
- receive as input a measured patient outcome;
- compare the predicted patient outcome with the received patient outcome to identify a discrepancy;
- in case of discrepancy, anonymize and store the measured patient outcome and the patient data (1) in a reference medical database.

9. The system according to claim **8,** wherein the patient predicted and measured outcomes are selected from a group comprising:
- future admissions or discharges;
- per-anesthetic outcomes;
- early or late post-anesthetic outcomes.

10. The system according to any of claims **5** to **9,** wherein the improvement algorithm is configured to:
- periodically, interrogate a medical guideline database about updated guidelines;
- modify or suggest a modification of the perianesthetic assessment algorithm based on the result of the interrogation.

11. The system according to any of claims **5** to **10,** wherein the improvement algorithm is a hybrid algorithm.

12. The system according to any of claims **5** to **11,** wherein the at least one sensor (12) is selected among: an optical sensor, a pressure sensor; a force sensor; a thermal sensor and the data collected from the at least one sensor (12) comprise physiological data or identity data.

13. The system according to any of claims **5** to **12,** further comprising an output module configured to:
- output a first pre-operative anesthesia evaluation comprising the index risk (2), the global anesthetic risk level (3) and the at least one recommendation (4);
- output a second pre-operative anesthesia evaluation comprising the selected at least one recommendation (4); information resulting from the user executing the at least one recommendation (4); the modified index risk and the modified global anesthetic risk level.

14. The system according to any of claims **5** to **13,** wherein the at least one patient index risk (2) is selected from a group consisting of: respiratory risk, neurological risk, kidney failure risk, immunologic risk, chronic pain risk, vascular risk, hepatic risk, cardiac risk, airway management risk, nausea and vomiting risk, thromboembolic risk, hemorrhagic risk.
